(19) Europäisches Patentamt

European Patent Office

Office européen des brevets

(11) Veröffentlichungsnummer: **0 513 862 A2**

# EUROPÄISCHE PATENTANMELDUNG

(21) Anmeldenummer: **92113162.9**

(22) Anmeldetag: **29.03.89**

(51) Int. Cl.⁵: **A61K  7/48**, A61K 31/20,
A61K 31/23, A61K 31/22

Diese Anmeldung is am 01 - 08 - 1992 als Teilanmeldung zu der unter INID-Kode 60 erwähnten Anmeldung eingereicht worden.

(30) Priorität: **30.05.88 DE 3811081**

(43) Veröffentlichungstag der Anmeldung:
**19.11.92 Patentblatt  92/47**

(60) Veröffentlichungsnummer der früheren Anmeldung nach Art. 76 EPÜ: **0 336 880**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen
Müllerstrasse 170/178 Postfach 65 03 11
W-1000 Berlin 65(DE)**

(72) Erfinder: **Nazzaro-Porro, Marcella, Dr.
Via Viminale 38
I-00184 Roma(IT)**

(54) **Verwendung von topisch applizierbaren Präparaten zur Behandlung der Altershaut.**

(57) Die Erfindung betrifft die Verwendung von topisch applizierbaren Präparaten enthaltend $\alpha,\omega$-n-Alkandicarbonsäuren mit 7 bis 13 Kohlenstoffatomen, deren physiologisch verträgliche Salze oder deren mittels Enzymen der Haut spaltbare Ester als Wirkstoff zur Behandlung der Altershaut.

EP 0 513 862 A2

Rank Xerox (UK) Business Services

Die Erfindung betrifft den in den Patentansprüchen gekennzeichneten Gegenstand.

Es ist bekannt, daß man topisch applizierbare Präparate, die als Wirkstoff $\alpha,\omega$-n-Alkandicarbonsäuren mit 7 bis 13 Kohlenstoffatomen, deren physiologisch verträgliche Salze oder deren mittels Enzymen der Haut spaltbare Ester enthalten, zur Behandlung einiger spezieller Erkrankungen der Haut verwenden kann. So wird in der US-Patentschrift 4,386,104 beschrieben, daß Präparate, die derartige Carbonsäuren als Wirkstoff enthalten zur Behandlung der Akne geeignet sind. In der Europäischen Patentanmeldung 0229654 wird beschrieben,daß sich derartige Präparate zur Behandlung entzündlicher und infektiöser Dermatosen eignen. In der deutschen Patentanmeldung 36 23 862 wird die Verwendung derartiger Präparate zur Behandlung der Rosazea beansprucht. Erwähnenswert scheint auch die US-Patentschrift 4,661,519, in der Mittel zur Behandlung der Akne beschrieben sind, die als Wirkstoffe mittels Enzymen der Haut leicht spaltbare Ester von a,$\omega$-n-Alkandicarbonsäuren mit 7 bis 13 Kohlenstoffatomen enthalten.

Es wurde nun gefunden, daß derartige Präparate überraschenderweise auch mit guten Erfolg zur Behandlung der Altershaut verwendet werden können. Unter dem Begriff Altershaut sollen erfindungsgemäß auch altersbedingte Veränderungen an den Hautanhanggebilden verstanden werden.

Bekanntlich neigt die Haut dazu, mit zunehmendem Alter trockner, faltiger, gelblicher, weniger elastisch und mechanisch weniger belastbar zu werden.

Mit zunehmendem Altar treten eine Vielzahl von pigmentierten und nicht pigmentierten Flecken an der Haut auf, wie zum Beispiel braune, graue oder gelbliche Altersflecken, Mitesser, Talgdrüsenvergrößerungen hartnäckige oder bleibende Gefäßerweiterungen insbesondere auch der oberflächlichen Hautgefäße und Hornhautverdickungen infolge langzeitiger Sonneneinwirkung. An den Füßen treten mit zunehmenden Alter infolge chronischer mechanischer Belastung sehr oft Hühneraugen, Schwielen und Nagelverkrümmungen auf.

Obgleich diese abnormalen Veränderungen der Haut von ihrer Genese her von den oben erwähnten Hauterkrankungen völlig verschieden sind, können sie überraschenderweise mit gutem Erfolg mit topisch applizierbaren Präparaten, enthaltend $\alpha,\omega$-n-Alkandicarbonsäuren mit 7 bis 13 Kohlenstoffatomen, deren physiologisch unbedenklichen Salzen oder deren mittels Enzymen der Haut spaltbaren Ester als Wirkstoffe behandelt werden, was mittels der vorbekannten Behandlungsweisen nicht in zufriedenstellendem Maße möglich war.

Es wurde bereits erwähnt, daß derartige topisch applizierbaren Präparate und ihre Herstellung vorbekannt sind.

Andererseits ist es aber auch möglich, neue, den speziellen Bedürfnissen der Altershaut angepaßte Zubereitungen zu erstellen (Am. Perfumer 77, 1962, 49; Z. Gerontol, 1976, 377; Drug Cosmet. Ind., 119, 1976, 54; Bristol Myers Nutr. Symp. 1986, 35).

Die Herstellung solcher topischen Zubereitungen erfolgt in üblicher Weise, indem man die Wirkstoffe mit geeigneten Zusätzen in die gewünschte Applikationsform, wie zum Beispiel eine Lösung, eine Milch, eine Lotion, eine Creme, eine Salbe, oder eine Paste überführt. In der so formulierten Zubereitung ist die Wirkstoffkonzentration von der Applikationsform abhängig. Vorzugsweise wird eine Wirkstoffkonzentration von 5 bis 30 Gewichtsprozent verwendet.

Die Milch, Lotion oder Creme (Öl/Wasser-Emulsionen) und die Salbe (Wasser/Öl-Emulsionen) kann in konventioneller Weise unter Verwendung konventioneller Emulgatoren hergestellt werden (Kirk Othmer: Enzyclopedia of Chemical Technology, 3. Auflage, 1979; John Wiley & Sons, New York, Vol. 8, Seiten 900 - 930, und Dr. Otto-Albrecht Neumüller: Römpps Chemie Lexikon, 7. Auflage, 1973; Franckh'sche Verlagshandlung Stuttgart, Seiten 1009 - 1013). Die für diese Emulsionen verwendeten Wachse, Emulgatoren und übrigen Zusätze sind die gleichen, wie man sie konventioneller Weise verwendet (Dr. Otto-Albrecht Neumüller: Römpps Chemie Lexikon, 7. Auflage, 1973; Franckh'sche Verlagshandlung Stuttgart, Seiten 1427 und 1428).

Die erfindungsgemäße topische Zubereitung kann aus hyxdrophilen und/oder lipophilen Wirkstoffen, Fettphase, Öl/Wasser-Emulgator, wäßriger Phase und Konservierungsmittel bestehen.

Als hydrophile und/oder lipophile Zusätze können Feuchthaltefaktoren (Hydrokomplexe), wie zum Beispiel Propylenglykol, Glycerin, Polyäthylenglykole, Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eignen sich Kohlenwasserstoffe, wie zum Beispiel Squalen, Vaseline, Paraffine oder Stearin, oder Wachse, wie zum Beispiel Bienenwachs oder tierische oder pflanzliche Öle, wie Olivenöl, Erdnußöl, feines Knochenöl, Mandelöl, Jojoba-Öl, Lanolin oder Sonnenblumenöl. Geeignete Öl/Wasser-Emulgatoren sind beispielsweise Stearylalkohol, Polyoxyäthylenstearate (wie zum Beispiel MYRJ[R]), Komplexemulgatoren (wie zum Beispiel Amphoterin[R]) und Sorbitanfettsäureester (wie zum Beispiel Tween 80[R]), Carboxyvinylpolymerisate (wie zum Beispiel Carbopol[R]), Fettalkohole wie zum Beispiel Cetylalkohol, Myristylalkohol oder Mische-

2

ster (wie zum Beispiel Dehymuls[(R)]). Die wäßrige Phase kann zusätzlich noch Puffersubstanzen, wie zum Beispiel das Dinatriumsalz der Äthylendiamin-N,N,N',N'-tetraessigsäure und Konservierungsmittel, wie Benzoesäure, Chlorquinaldol, Parabene oder Benzalkoniumchlorid, enthalten.

Die Emulsion wird zusätzlich mit dem mikronisierten Wirkstoff (Korngröße vorzugsweise 1 bis 20 $\mu$) und gegebenenfalls noch mit Duftstoffen, wie zum Beispiel diejenige der Crematest[(R)]-Serie, versetzt und bis zur gleichmäßigen Verteilung derselben gerührt.

Als Wirkstoffe werden $\alpha,\omega$-n-Alkandicarbonsäuren mit 7 bis 13 Kohlenstoffatomen, deren physiologisch verträgliche Salze oder deren mittels Enzymen der Haut spaltbare Ester eingesetzt.

Zu den erfindungsgemäß verwendeten Dicarbonsäuren gehören insbesondere Pimelinsäure, Suberinsäure, Azelainsäure, Sebazinsäure, 1,9-Nonandicarbonsäure, 1,10-Decandicarbonsäure und 1,11-Undecandicarbonsäure.

Zu den mittels Enzymen der Haut spaltbaren Estern der Dicarbonsäuren zählen beispielsweise die 2,3-Dihydroxy-propyloxyester dieser Substanzen.

Zu den physiologisch verträglichen Salzen zählen Alkalimetallsalze, wie Natrium- und Kaliumsalze, ferner Salze mit basischen Aminoverbindungen und organischen Aminen, wie zum Beispiel Arginin, Lysin oder N-Methylglucamin.

In einer bevorzugten Ausführungsform wird Azelainsäure als Dicarbonsäure verwendet.

Es ist oft vorteilhaft, den erfindungsgemäßen Mitteln zusätzlich noch ca. 1 bis 4 Gewichtsprozent einer keratolytisch wirksamen Substanz, wie zum Beispiel Salicylsäure oder Resorcin - bezogen auf das Gesamtgewicht des Mittels - zuzusetzen.

Das nachfolgende Ausführungsbeispiel dient zur näheren Erläuterung der Erfindung.

Beispiel

17 Patienten mit verschiedenen altersbedingten Veränderungen der Gesichtshaut und 4 Patienten mit altersbedingten Anormalien der Extremitäten wurden zwei mal pro Tag mit einer Creme behandelt, enthaltend

|  | Gewichtsprozent |
|---|---|
| Azelainsäure | 20,0 % |
| Benzoesäure | 0,1 % |
| Salizylsäure | 2,0 % |
| Ascorbinsäure | 1,0 % |
| Glycerin monostearat | 2,0 % |
| Cetylalkohol | 3,0 % |
| Polyentyhlen(20)sorbitanmonoaleat | 5,0 % |
| Natriumlaurylsulfat | 10,0 % |
| Ethanolamin-laurylsulfat | 1,0 % |
| Olivenöl | 2,0 % |
| Bidestilliertes Wasser | 53,9 % |
| -hergestellt gemäß DE-A 36 23 862- | |

Nach 6 Monaten Behandlungsdauer wurde die Creme nur einmal pro Tag oder sporadisch über einen langen Zeitraum aufgetragen.

Zwecks Dokumentation des Behandlungsergebnisses wurden die behandelten Körperpartien vor, während und nach der Behandlung fotografiert.

In der nachfolgenden Tabelle sind nähere Angaben über die behandelten Patienten aufgeführt.

Tabelle

| Patient Nr. | Initialen des Patienten | Behandlungsbeginn | Alter bei Behandlungsbeg. | Geschlecht | Letzte Behandlung |
|---|---|---|---|---|---|
| 1 | Z.M. | 21.11.77 | 55 | F | 9.10.87 |
| 2 | R.M. | 8.10.82 | 70 | F | 14.01.88 |
| 3 | B.A. | 9.10.80 | 81 | F | 19.12.80 |
| 4 | C.M. | 5.01.84 | 63 | F | 18.01.88 |
| 5 | D.P. | 9.06.87 | 49 | F | 18.01.88 |
| 6 | S.T. | 24.05.85 | 66 | F | 12.01.88 |
| 7 | B.C. | 4.03.85 | 58 | M | 12.01.88 |
| 8 | O.Q. | 27.10.83 | 66 | M | 20.01.88 |
| 9 | M.M. | 29.05.85 | 71 | F | 09.10.87 |
| 10 | F.M. | 10.05.83 | 56 | F | 12.01.88 |
| 11 | G.L. | 14.05.87 | 65 | M | 11.02.88 |
| 12 | L.L. | 19.09.86 | 54 | F | 12.01.88 |
| 13 | S.C. | 24.05.85 | 65 | F | 29.01.88 |
| 14 | B.P. | 12.03.85 | 59 | M | 11.02.88 |
| 15 | D.P. | 8.11.83 | 72 | M | 10.12.87 |
| 16 | G.G. | 27.06.85 | 79 | M | 11.02.88 |
| 17 | D.P. | 20.04.85 | 63 | M | 9.02.88 |
| 18 | M.V. | 26.11.85 | 48 | F | 12.02.88 |
| 19 | Z.F. | 10.09.82 | 64 | F | 10.12.87 |
| 20 | P.C. | 26.06.82 | 56 | M | 29.07.87 |
| 21 | A.M. | 22.09.87 | 61 | F | 18.01.88 |

M = männlich F = weiblich

Die Patienten 1 bis 4 litten an einer Lentigo maligna. Es zeigte sich, daß nicht nur bei dieser Erkrankung eine Abheilung erzielt werden konnte, sondern daß auch nicht betroffene Areale der Haut, wenn sie mit der Creme regelmäßig behandelt wurden, im Vergleich zu unbehandelten Partien der Haut sehr deutlich verbesserte Beschaffenheit zeigten, sie waren nach der Behandlung wesentlich elastischer, glatter, weniger faltig und erschienen nicht mehr gelblich sondern rosafarben. Durch Sonneneinwirkung verursachte Verhornungen (Keratosen) und andere Flecken waren nach erfolgter Behandlung ganz oder fast völlig beseitigt.

Die Patienten 5 bis 11 hatten vor der Behandlung Hautareale mit hartnäckigen Gefäßerweiterungen insbesondere solche der oberflächennahen Hautgefäße (altersbedingte Vasodilatationen und Teleangiektasien). Auch diese Hautanomalien konnten durch die Behandlung ganz oder überwiegend beseitigt werden. Zusätzlich konnten bei den Patienten 8 und 11 große Mitesser (Komedos) und Talgdrüsenerweiterungen (Talgdrüsenhyperplasien) durch die Behandlung entfernt werden. Gute bis sehr gute Behandlungsergebnisse wurden auch bei solchen Patienten erzielt, die durch langzeitige Sonnenexposition verursachte Keratosen (Patienten 12 bis 16), altersbedingte seborrhoischen Dermatosen (17), Schwielen (Patient 18) auf der Hand und am Fuß befindliche Ekzeme (Patienten 18 und 19) sowie eine Verkrümmung der Fußnägel (Patienten 20 und 21) hatten.

Die bei den Patienten 1 bis 4 beobachtete sehr deutlich verbesserte Beschaffenheit der behandelten Hautpartien wurde auch bei den übrigen Patienten beobachtet, deren behandelte Haut "verjüngt" zu sein schien.

**Patentansprüche**

1. Verwendung von topisch applizierbaren Präparaten enthaltend $\alpha,\omega$-n-Alkandicarbonsäuren mit 7 bis 13 Kohlenstoffatomen, deren physiologisch verträgliche Salze oder deren mittels Enzymen der Haut spaltbare Ester als Wirkstoff zur Behandlung der Altershaut.

2. Verwendung von topisch applizierbaren Präparaten gemäß Patentanspruch 1 zur Behandlung der Altershaut,
dadurch gekennzeichnet, daß diese Präparate in flüssiger oder halbfester Form vorliegen.

**3.** Verwendung von topisch applizierbaren Präparaten gemäß Patentanspruch 1 und 2 zur topischen Behandlung der Altershaut,
dadurch gekennzeichnet, daß diese Präparate 5 bis 30 Gewichtsprozent Dicarbonsäure, Dicarbonsäuresalz oder Dicarbonsäureester als Wirkstoff enthalten.

**4.** Verwendung von topisch applizierbaren Präparaten gemäß Patentanspruch 1 bis 3 zur topischen Behandlung der Altershaut,
dadurch gekennzeichnet, daß diese Präparate 1,7-Heptandicarbonsäure deren physiologisch verträgliche Salze oder deren mittels Enzymen der Haut spaltbare Ester als Wirkstoff enthalten.

**5.** Verwendung von $\alpha,\omega$-n-Alkandicarbonsäuren mit 7 bis 13 Kohlenstoffatomen, deren physiologisch verträglichen Salze oder deren mittels Enzymen der Haut spaltbaren Ester zur Herstellung von topisch applizierbaren Präparaten zur Behandlung der Altershaut.